# FASCICULE DE BREVET EUROPEEN

(11) **EP 1 471 944 B1**
(45) Date de publication et mention de la délivrance du brevet: **24.05.2006**
(21) Numéro de dépôt: 03718854.7
(22) Date de dépôt: 07.02.2003
(51) Int. Cl.: A61K 48/00, A61K 31/77, A61K 31/785, A61P 43/00

(54) **COMPOSITION PHARMACEUTIQUE AMELIORANT LE TRANSFERT DE GENE IN VIVO**
PHARMAZEUTISCHE ZUSAMMNESETZUNG ZUR VERBESSERTEN IN-VIVO GENÜBERTRAGUNG
PHARMACEUTICAL COMPOSITION WHICH IMPROVES IN VIVO GENE TRANSFER

(30) Priorité: 08.02.2002 FR 0201584
(43) Date de publication de la demande: 03.11.2004
(73) Titulaire: INSTITUT NATIONAL DE LA SANTE ET DE LA RECHERCHE MEDICALE (INSERM), 75013 Paris (FR)
(72) Inventeur: PITARD, Bruno, F-44400 Reze (FR)
(74) Mandataire: Le Coupanec, Pascale A.M.P.
(86) Numéro de dépôt international: PCT/FR2003/000407
(87) Numéro de publication internationale: WO 2003/066104

(56) Documents cités:
- WO-A-00/47186
- WO-A-00/51645
- WO-A-01/08709
- WO-A-01/60415
- WO-A-01/65911
- WO-A-93/15745
- WO-A-96/15778
- WO-A-96/21470
- WO-A-99/31262
- WO-A-02/061040
- US-B1- 6 221 959
- KLEBE R J ET AL: "UPTAKE BY CELLS OF NUCLEIC ACIDS PROMOTED BY COMPOUNDS SHARING THE PLEIOTROPIC EFFECTS OF POLY(ETHYLENE GLYCOL)" TERATOGENESIS, CARCINOGENESIS, MUTAGENESIS, ALAN R. LISS, NEW YORK, NY, US, vol. 6, no. 3, 1986, pages 245-250, XP000572777 ISSN: 0270-3211
- PROKOP ALES ET AL: "Maximizing the in vivo efficiency of gene transfer by means of nonviral polymeric gene delivery vehicles." JOURNAL OF PHARMACEUTICAL SCIENCES, vol. 91, no. 1, janvier 2002 (2002-01), pages 67-76, XP002253712 ISSN: 0022-3549
- KAKIZAWA Y ET AL: "BLOCK COPOLYMER MICELLES FOR DELIVERY OF GENE AND RELATED COMPOUNDS" ADVANCED DRUG DELIVERY REVIEWS, AMSTERDAM, NL, vol. 54, 2002, pages 203-222, XP001150717 ISSN: 0169-409X

## Description

La présente invention vise à titre principal une composition pharmaceutique permettant de faciliter le transfert cellulaire d'acide(s) nucléique(s) et plus particulièrement dans des cellules musculaires ou cardiaques *in vivo.*

La problématique considérée dans le cadre de la présente invention est celle du transfert d'une information génique dans une cellule et plus particulièrement dans son noyau, afin que le gène considéré y soit traduit en protéine.

Actuellement, deux technologies sont principalement mises en oeuvre pour le transfert cellulaire de gène. La première met en oeuvre l'adeno associated virus, AAV, qui est un virus recombinant. Quant à la seconde, il s'agit de l'électrotransfert qui consiste à imposer un champ électrique au niveau des cellules considérées après y avoir injecté l'ADN. Bien que ces deux méthodes permettent d'obtenir des niveaux satisfaisants d'efficacité de transfert de gènes, elles présentent en revanche un inconvénient en termes de toxicité. Qui plus est, l'AAV soulève aujourd'hui de nombreuses difficultés techniques pour sa préparation, purification et/ou caractérisation et le risque potentiel d'intégration dans le génome de la cellule hôte pourrait faire encourir des phénomènes de cancérisation. Enfin, le transfert de gènes dans le muscle cardiaque n'est pas accessible par la technique de l'électrotransfert pour des raisons de survie.

En l'occurrence, la présente invention est précisément avantageuse pour la transfection dans des cellules de muscles squelettiques, lisses et cardiaques.

En ce qui concerne les vecteurs synthétiques, à l'image des lipides cationiques par exemple, ils s'avèrent inefficaces *in vivo*. Seul l'ADN nu est capable de conduire à l'expression d'une protéine après injection dans le muscle squelettique et cardiaque. Malheureusement, la quantité de protéine synthétisée, après injection intramusculaire d'ADN nu, demeure insuffisante pour envisager des applications cliniques. En effet, un des problèmes majeurs de l'utilisation de l'ADN nu pour le transfert de gène dans les tissus musculaires est sa faible efficacité, que n'améliore pas l'augmentation de quantités d'ADN injecté. Or, pour des raisons de commodité, notamment en terme d'accessibilité, il serait particulièrement intéressant de privilégier l'expression de protéines d'intérêt thérapeutique local ou systémique au niveau de ces muscles.

En conséquence, il existe aujourd'hui un besoin réel d'un vecteur idéal en thérapie génique, c'est-à-dire un vecteur non toxique, n'induisant pas de réaction immunitaire et permettant d'obtenir une expression optimale en protéine dont l'effet thérapeutique est recherché.

La présente invention a précisément pour objet de proposer l'utilisation de molécules chimiques spécifiques pour accroître significativement l'efficacité de transfection de l'ADN auquel elles sont associées.

Plus précisément, la présente invention concerne une composition pharmaceutique caractérisée en ce qu'elle associe à au moins un acide nucléique, au moins un sel minéral du copolymère tétrafonctionnel de formule (I): 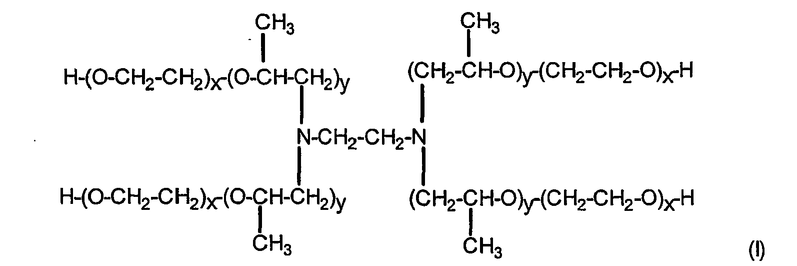 dans laquelle x et y représentent indépendamment l'un de l'autre un entier compris entre 1 et 500 avec x possédant une valeur telle que ladite molécule comprend au moins 30 % en poids de motifs oxyde d'éthylène,
la dite composition étant formulée avec du milieu Tyrode, à savoir 140 mM NaCl, 6 mM KCI, 3 mM CaCl₂, 2 mM MgCl₂, 10 mM Hepes pH 7.4 et 10 mM glucose (Tyrode Pharmacology. Philadelphia, 1908, 2^{nd} Edition, 1912).

Les molécules précitées, qui sont des poloxamines particulières, sont constituées de segments hydrophobes (oxyde de propylène, portant les indices y), de segments hydrophiles (oxyde d'éthylène, portant les indices x) et d'une partie centrale éthylène-diamine chargée positivement (NCH₂ - CH₂ N).

Les inventeurs ont plus particulièrement mis en évidence que ces poloxamines, qui étaient jusqu'alors présentées comme des polymères non-ioniques (WO 00/47 186) peuvent en fait prendre une forme cationique.

De manière surprenante, l'apparition de ces charges positives au niveau de la partie centrale éthylène-diamine n'entame en rien l'efficacité du transfert cellulaire, au contraire. En effet, les molécules chimiques cationiques de type lipides cationiques, proteines et peptides cationiques n'améliorent pas, par rapport à l'ADN nu le transfert de gène dans les tissus musculaires (Lluis M.M. et al., 1999, *Proc*. *Natl. Acad. Sci*., vol. 96, pp. 4262-4267).

Les demandes de brevets WO 01/65911 A, WO 93/15745 A, WO 00/51645 A, WO 96/21470 A, ainsi que Prokop et al. (J. Pharm. Sci. 2002, vol. 91, no. 1, pp. 67-76) divulguent l'utilisation de poloxamères et/ou de poloxamines, telles que les Tetronics® pour le transfert intracellulaires *in vitro* ou *in vivo* de gènes et d'acides nucléiques, présentant éventuellement une biodisponibilité améliorée, notamment à des fins thérapeutiques.
Klebe et al. (Teratogenesis, Carcinogenesis, Mutagenesis 1986, vol. 6, no. 3, pp. 245-250) décrit l'utilisation de dérivés du polyéthylène glycol tel que le Tetronics® 304 polyol en combinaison avec une solution tampon TES pour la transformation génétique de cellules au moyen d'un ARN viral.
La demande de brevet WO 96/15778 A et le brevet US 6221959 B divulguent des compositions comprenant des polyoxamines de type Tetronic® à titre d'agents actifs pour stabiliser des polynucléotides pour la tranfection de cellules.

Un objet particulier de l'invention est donc une composition pharmaceutique, caractérisée en ce qu'elle associe à au moins un acide nucléique au moins un sel minéral du copolymère tétrafonctionnel de formule (I): 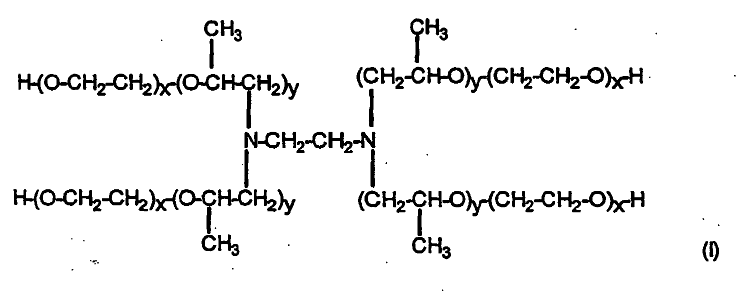 mis en oeuvre sous une forme cationique
dans laquelle x et y représentent indépendamment l'un de l'autre un entier compris entre 1 et 500 avec x possédant une valeur telle que ladite molécule comprend au moins 30 % en poids de motifs oxyde d'éthylène,
la dite composition étant formulée avec du milieu Tyrode, à savoir 140 mM NaCl, 6 mM KCl, 3 mM CaCl₂, 2 mM MgCl₂, 10 mM Hepes pH 7.4 et 10 mM glucose. La présence du Tyrode permet notamment le contrôle de la composition ionique de la formulation et du pH et, par conséquent, de la mise en oeuvre sous une forme cationique du composé de formule (I).

Les inventeurs ont en outre établi l'intérêt de contrôler le pH et/ou la composition ionique de la formulation, afin de s'assurer que le copolymère de formule (I) soit sous sa forme cationique.

Un pH de 6,5 à 8, de préférence 7 à 7,8, de préférence encore 7,4,

Sans vouloir se lier à un quelconque mécanisme d'action, les inventeurs observent en fait que les poloxamines mises en oeuvre sous une forme cationique en présence du milieu Tyrode, permettent de condenser l'ADN.

De manière inattendue, les inventeurs ont en outre mis en évidence que les composés de formule générale (I), dans laquelle les motifs oxyde d'éthylène sont présents à raison d'au moins 30 % en poids, s'avèrent particulièrement efficaces pour le transfert d'un gène associé *in vivo*. Cette efficacité est en particulier illustrée par l'exemple 1 ci-après. Selon une variante préférée, les composés selon l'invention comprennent au plus 85 % en poids de motifs oxyde d'éthylène. Plus préférentiellement, ils possèdent entre 35 et 50 % et de préférence 40 % en poids de motifs oxyde éthylène.

Selon une variante préférée de l'invention, les molécules de composés de formule générale (I) possèdent en outre un poids moléculaire d'au moins 800 g/mol. et plus préférentiellement compris entre 1 000 et 25 000 g/mol.

Selon un mode de réalisation préféré de l'invention, les composés de formule générale possèdent un rapport en motifs OE/OP compris entre 0,5 et 1,5 et de préférence de l'ordre de 1 ± 0,2.

A titre de composés de formule générale (I) convenant tout particulièrement à la présente invention, on peut plus particulièrement citer les molécules possédant respectivement un poids moléculaire de 1 650 g pour un rapport OE/OP 15 :16 (par exemple la poloxamine 304), de 5 500 g/mol. pour un rapport OE/OP 50:56 (par exemple la poloxamine 704) et de 6 700 g/mol. pour un rapport OE/OP 61:68 (par exemple la poloxamine 904).

Au sens de la présente invention, le terme acide nucléique couvre aussi bien un acide désoxyribonucléique qu'un acide ribonucléique.

En l'occurrence, il peut s'agir de séquences d'origine naturelle ou artificielle, et notamment d'ADN génomique, d'ADNc, d'ARNm, d'ARNt, d'ARNr, d'ARN interférent (ARNi ou small interférence RNA), de séquences hybrides ou de séquences synthétiques ou semi-synthétiques d'oligonucléotides modifiées ou non. Ces acides nucléiques peuvent être d'origine humaine, animale, végétale, bactérienne, virale, etc. Ils peuvent être obtenus par toute technique connue de l'homme du métier, et notamment par criblage de banques, par synthèse chimique ou encore par des méthodes mixtes incluant la modification chimique ou enzymatique de séquences obtenues par criblage de banques. Ils peuvent être modifiés chimiquement.

Concernant plus particulièrement les acides désoxyribonucléiques, ils peuvent être simple ou double brins, de même que des oligonucléotides courts ou des séquences plus longues. Ces acides désoxyribonucléiques peuvent porter des gènes thérapeutiques, des séquences régulatrices de la transcription ou de la réplication, des séquences antisens modifiées ou non, des régions de liaison à d'autres composants cellulaires, etc. Ils peuvent notamment diriger la synthèse d'un polypeptide spécifique d'un agent infectieux ou être capable de remédier à une déficience génétique ou acquise.

Au sens de l'invention, on entend par gène thérapeutique notamment tout gène codant pour un produit protéique ayant un effet thérapeutique. Le produit protéïque ainsi codé peut être une protéine, un peptide, etc. Ce produit protéïque peut être homologue vis-à-vis de la cellule cible (c'est-à-dire un produit qui est normalement exprimé dans la cellule cible lorsque celle-ci ne présente aucune pathologie). Dans ce cas, l'expression d'une protéine permet, par exemple, de pallier une expression insuffisante dans la cellule ou l'expression d'une protéine inactive ou faiblement active en raison d'une modification, ou encore de surexprimer ladite protéine. Le gène thérapeutique peut aussi coder pour un mutant d'une protéine cellulaire, ayant une stabilité accrue, une activité modifiée, etc. Le produit protéïque peut également être hétérologue vis-à-vis de la cellule cible. Dans ce cas, une protéine exprimée peut, par exemple, compléter ou apporter une activité déficiente dans la cellule, lui permettant de lutter contre une pathologie ou simuler une réponse immunitaire.

Parmi les produits thérapeutiques au sens de la présente invention, on peut citer plus particulièrement les enzymes, les dérivés sanguins, les hormones, les lymphokines comme les interleukines, interférons, TNF, etc., les facteurs de croissance comme le facteur de croissance vasculaire endothéliale, le facteur de croissance insulin-like et le facteur de croissance fibroblaste, les neurotransmetteurs ou leurs précurseurs ou enzymes de synthèse, les facteurs trophiques tels que BDNF, CNTF, NGF, IGF, GMF, aFGF, bFGF, NT3, NT5 et HARP/pléiotrophine, la dystrophine ou une minidystrophine, l'utrophine, la protéine CFTR associée à la mucoviscidose, les gènes supresseurs de tumeurs comme p53, Rb, Rap1A, DCC et k-rev, les gènes codant pour des facteurs impliqués dans la coagulation de type facteurs VII, VIII et IX, les gènes intervenant dans la réparation de l'ADN, les gènes suicides (thymidine kinase, cytosine déaminase), les gènes de l'hémoglobine ou d'autres transporteurs protéïques, les gènes correspondant aux protéines impliquées dans le métabolisme des lipides, de type apolipoprotéine choisie parmi les apolipoprotéines A-I, A-II, A-IV, B, C-I, C-II, C-III, D, E, F, G, H, J et Apo(A), les enzymes du métabolisme comme, par exemple, la lipoprotéine lipase, la lipase hépatique, la lécithine cholestérol acyltransférase, la 7 alpha cholestérol hydroxylase, la phosphatidique acide phosphatase ou encore des protéines de transfert de lipides comme la protéine de transfert des esters de cholestérol et la protéine de transfert des phospholipides, une protéine de liaisons des HDL ou encore un récepteur choisi, par exemple, parmi les récepteurs LDL, récepteurs des chylomicrons-remnants et les récepteurs scavenger, l'érythropoïétine, la protéinekinase C-3, etc.

L'acide nucléique thérapeutique peut également être un gène ou une séquence antisens, dont l'expression dans la cellule cible permet de contrôler l'expression de gènes ou la transcription d'ARNm cellulaires. De telles séquences peuvent, par exemple, être transcrites dans la cellule cible en ARN complémentaires d'ARNm cellulaires et bloquer ainsi leur traduction en protéine. Les gènes thérapeutiques comprennent également les séquences codant pour des ribozymes, qui sont capables de détruire sélectivement des ARN cibles.

Comme indiqué plus haut, l'acide nucléique peut également comporter un ou plusieurs gènes codants pour un peptide antigénique, capable de générer chez l'homme ou l'animal une réponse immunitaire. Dans ce mode particulier de mise en oeuvre, l'invention permet donc la réalisation soit de vaccins, soit de traitements immunothérapeutiques appliqués à l'homme ou à l'animal, notamment contre des microorganismes, des virus ou des cancers. II peut s'agir notamment de peptides antigéniques spécifiques du virus d'Epstein Barr, du virus HIV, du virus de l'hépatite B, du virus de la pseudo-rage, du « syncitia forming virus », d'autres virus ou encore spécifiques de tumeurs.

Préférentiellement, l'acide nucléique comprend également des séquences permettant l'expression du gène thérapeutique et/ou du gène codant pour le peptide antigénique dans la cellule ou l'organe désiré. Il peut s'agir des séquences qui sont naturellement responsables de l'expression du gène considéré lorsque ces séquences sont susceptibles de fonctionner dans la cellule infectée. Il peut également s'agir de séquences d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus. A cet égard, on peut citer par exemple les promoteurs des gènes E1A, MLP, CMV, RSV, et les promoteurs tissus spécifiques comme les promoteurs de chaînes de myosine par exemple, etc. En outre, ces séquences d'expression peuvent être modifiées par addition de séquences d'activation, de régulation, etc. Il peut aussi s'agir de promoteur, inductible ou répressible.

Par ailleurs, l'acide nucléique peut également comporter, en particulier en amont du gène thérapeutique, une séquence signal dirigeant le produit thérapeutique synthétisé dans les voies de sécrétion de la cellule cible. Cette séquence signal peut être la séquence signal naturelle du produit thérapeutique, mais il peut également s'agir de toute autre séquence signal fonctionnelle, ou d'une séquence signal artificielle. L'acide nucléique peut également comporter une séquence signal dirigeant le produit thérapeutique synthétisé vers un compartiment particulier de la cellule.

Outre le composé de formule générale (I), les compositions revendiquées peuvent comprendre un ou plusieurs adjuvant(s) et notamment un tensioactif.

A titre représentatif de ces adjuvants, on peut plus particulièrement citer les celluloses, comme le carboxylméthylcellulose, hydroxypropylcellulose, les sels hyaluronate ou alginate, les pectines, les polyéthylènes glycol, les dextrans, les polyvinyles pyrolidones, les chitosants, les alcools polyvinyliques, les propylènes glycol, les acétates de polyvinyle, les lécithines, les acides polylactiques et polyhydroxybutyriques et les poloxamères de la série des pluronics® (POE-PPO-POE) et pluronics® inverses (PPO-POE-PPO).

Les compositions selon l'invention peuvent également mettre en oeuvre un ou plusieurs éléments de ciblage permettant de diriger les complexes nucléiques vers des récepteurs ou des ligands à la surface de la cellule. A titre d'exemple, la composition de la présente invention peut comprendre un ou plusieurs anticorps dirigés contre des molécules de la surface cellulaire, ou encore un ou plusieurs ligands de récepteurs membranaires comme l'insuline, la transferrine, l'acide folique ou tout autre facteur de croissance, cytokines ou vitamines. Avantageusement, la composition peut utiliser des lectines, modifiées ou non, afin de cibler des polysaccharides particuliers à la surface de la cellule ou sur la matrice extracellulaire avoisinante. Des protéines à motif RGD, des peptides contenant un tandem de motifs RGD, cyclique ou non, ainsi que des peptides polylysine ou des peptides ligands, naturels ou synthétiques peuvent ainsi être utilisés.

Le composé de formule générale (I) peut être incorporé à raison de 0,005 % à 15 % en poids/volume de ladite composition et plus préférentiellement entre 0,01 % et 10 % en poids/volume.

Les compositions revendiquées sont obtenues par mélange de l'acide nucléique considéré avec au moins un composé de formule générale (I) au sein d'une solution compatible avec une administration *in vivo*. A titre illustratif, ces compositions peuvent être préparées à l'aide du protocole suivant : mélange, volume à volume, d'une solution contenant l'acide nucléique (notamment ADN) concentré deux fois dans 300 mM NaCl ou du Tyrode 2X (deux fois concentré) et d'une solution aqueuse contenant un composé de formule générale (I) deux fois plus concentrée. L'ensemble est agité, de préférence par VORTEX.

Il a ainsi été constaté que les composés de formule générale (I) revendiqués permettent d'augmenter la quantité de protéine synthétisée par le muscle d'un facteur allant de 5 à 20 comparativement à l'ADN nu. Par ailleurs, il n'a pas été noté avec la composition suivant l'invention d'expression d'un transgène dans des cellules en culture *in vitro,* en utilisant les méthodes classiques de culture de cellules.

De préférence, les compositions pharmaceutiques de l'invention contiennent un véhicule pharmaceutiquement acceptable pour une formulation injectable, notamment pour l'injection directement au niveau de l'organe désiré ou pour une administration par voie topique, par exemple sur peau et/ou muqueuses. Il peut s'agir en particulier de solutions stériles, isotoniques ou de compositions sèches, notamment lyophilisées, qui, par addition selon le cas d'eau stérilisée ou de sérum physiologique, permettent la constitution de solutés injectables.

Il est clair que les doses d'acide nucléique utilisées pour l'injection ainsi que le nombre d'administrations peuvent être adaptés de différents paramètres, et notamment en fonction du mode d'administration considéré, de la pathologie concernée, de la nature du gène à exprimer ou encore de la durée de traitement recherchée.

En ce qui concerne plus particulièrement le mode d'administration, il peut s'agir soit d'une injection directe dans les tissus ou les voies circulatoires, soit d'un traitement de cellules en culture suivi de la réimplantation *in vivo* par injection ou greffe.

La composition revendiquée s'avère particulièrement avantageuse pour une administration interne.

Au sens de la présente invention, administration interne signifie que les compositions revendiquées sont compatibles avec une administration dans le tissu d'un organisme, par exemple un muscle, par voie intradermique ou sous-cutanée. D'autre part, sont susceptibles d'être utilisées les voies topique, orale, pulmonaire, nasale et mycosale, comme par exemple buccale, vaginale ou rectale.

Les compositions selon l'invention sont particulièrement avantageuses sur le plan thérapeutique.

C'est ainsi que les applications potentielles sont dans le domaine de la thérapie génique et notamment dans la production par un tissu musculaire d'une protéine d'intérêt thérapeutique, local ou systémique.

En effet, après transfert de gènes dans le muscle, en présence d'au moins une molécule de formule générale (I), cet organe peut servir de réservoir de synthèse de protéines hétérologues qui vont agir soit au niveau local (facteur angiogénique...) ou systémique (facteur de coagulation, de croissance, insuline...).

Qui plus est, la composition revendiquée peut permettre d'abolir l'effet plateau obtenu avec l'ADN nu. En effet, lorsque la quantité d'ADN injecté dans le muscle augmente, la transfection augmente linéairement jusqu'à une certaine dose, puis la quantité de protéine exprimée plafonne. En revanche, avec des formulations conformes à l'invention, la quantité de protéine exprimée augmente exponentiellement avec l'augmentation de la quantité d'ADN injecté dans le muscle.

Une autre application relève du domaine de la vaccination. En l'occurrence, on injecte dans des cellules, de préférence musculaires, un ADN codant pour un antigène bactérien, viral ou autre. Dans la mesure où les compositions revendiquées sont particulièrement avantageuses pour augmenter la quantité de protéine synthétisée par les cellules transfectées, il est possible, par ce biais, d'obtenir des concentrations plus importantes en anticorps et en lymphocytes T cytotoxiques.

La présente invention a également pour objet l'utilisation d'un composé de formule générale I ou d'un de ses sels organique ou minéral à titre de vecteur pour le transfert cellulaire *in vivo* d'au moins un acide nucléique tel que défini selon l'invention.

Est également du domaine de l'invention l'utilisation d'un composé de formule générale I tel que défini ci-dessus pour la préparation d'une composition destinée à assurer le transfert cellulaire *in vivo* d'au moins un acide nucléique.

L'invention concerne également une méthode de transfection cellulaire *in vivo* d'au moins un acide nucléique caractérisée en ce que l'on administre ledit acide nucléique conjointement avec au moins un composé de formule générale I tel que défini ci-dessus ou un de ses sels organique ou minéral.

L'administration peut être réalisée par voie topique, directement dans les cellules considérées ou selon une des voies d'administrations discutées ci-dessus.

Selon une variante privilégiée de l'invention les cellules cibles sont des cellules musculaires ou cardiaques.

Selon un mode préféré de l'invention, on utilise la poloxamine 304, à titre de vecteur pour transférer un acide nucléique *in vivo* dans les cellules musculaires, ou surtout cardiaques.

La poloxamine 304 est formulée au sein d'une composition contenant du milieu Tyrode.

La présente invention sera mieux décrite à l'aide des exemples et figures qui suivent, qui doivent être considérés comme illustratifs et non limitatifs.

Les formulation formulée avec du NaCl ne font pas partie de l'invention brevetée et ont été mentionnées dans les exemples à titre comparatif.

### FIGURES

Figure 1 : Expression de la luciferase après injection intramusculaire d'ADN formulé selon l'invention.
Figure 2 : Visualisation macroscopique de l'activité β-galactosidase après injection, dans le tibial anterior de souris, d'ADN formulé selon l'invention.
Figure 3 : Visualisation par microscopie de fluorescence l'expression de GFP dans une cellule de muscle.
Figure 4 : Représentation de l'expression de β galactosidase dans les libial anterior de souris après injection par électro transfert, dudit ADN ou formulé selon l'invention.
Figure 5 : Visualisation par cryo-microscopie électronique. La poloxamine 904 à 10% est mélangée avec de l'ADN à 0,15 mg/ml soit dans 150 mM NaCl (A,B) soit dans du Tyrode (140 mM NaCl, 6mM KCl, 3 mM CaCl₂, 2mM MgCl₂, 10 mM Hepes pH7.4 et 10 mM glucose) (C, D, E). Barre d'échelle 100 nm.

### Matériels :

Les composés de formule générale (I) testés sont référencés ci-après dans le tableau 1 qui rend compte de leurs caractéristiques physico-chimiques.

**Tableau 1**

| Molécule poloxamine | MW (g/mol) | % oxyde d'éthylène | Nombre d'oxyde d'éthylène | Nombre d'oxyde de propylène | OE/OP |
|---|---|---|---|---|---|
| 304 | 1650 | 40 | 15 | 16 | 0,93 |
| 704 | 5500 | 40 | 50 | 56 | 0,89 |
| 904 | 6700 | 40 | 61 | 68 | 0,89 |
| 908 | 25000 | 80 | 454 | 85 | 5,3 |

### Exemple 1 :

### Transfection in vivo dans le muscle d'un gène en présence d'un composé de formule générale (I)

### • Préparation des échantillons :

Deux concentrations optimisées pour chacun des produits testés ont été considérées.

Chaque essai met en oeuvre 15 µg d'ADN dans 50 µl de NaCl 150 mM associé à deux concentrations différentes d'un composé de formule générale (I) testée.

Les concentrations testées sont pour le composé 904 respectivement 0,01 % et 0,1 %, pour le composé 704 : 0,25% et 0,5% et pour le composé 304 : 5 % et 10 %.

Un échantillon témoin d'ADN est formulé en l'absence de composés de formule générale (I).

Chaque échantillon ainsi formulé est injecté dans le tibial anterior de souris swiss âgées de sept semaines. Après sept jours, les souris sont sacrifiées et le tibial anterior injecté est disséqué puis broyé dans un tampon de lyse en présence d'un cocktail d'inhibiteurs de protéases de Roche Diagnostics. L'activité luciférase est dosée dans le surnageant par luminométrie (Vector 2 de Perkin Elmer, Les Ulis, France) en utilisant le kit de dosage Luciferase Assay System® distribué par Promega (Charbonnières, France).

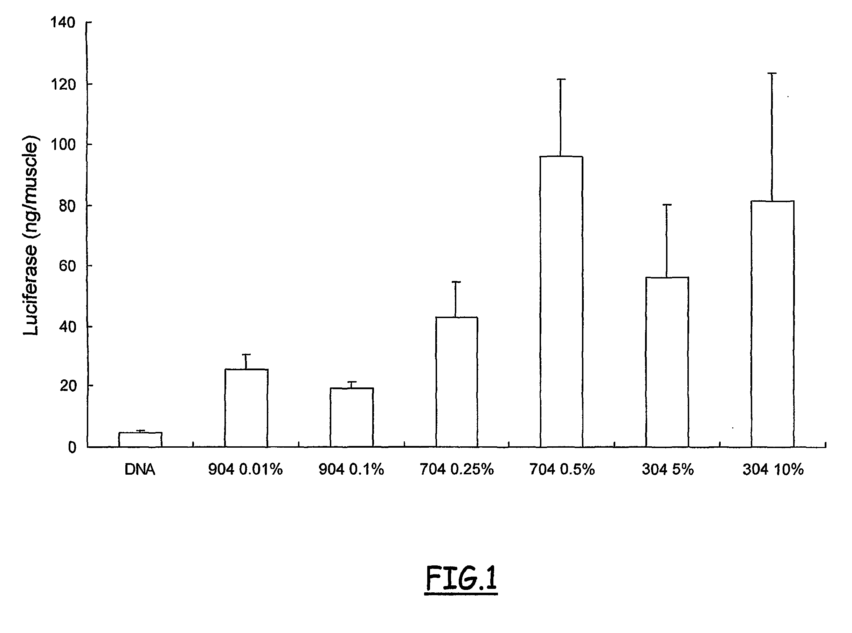

La figure 1 rend compte des résultats obtenus.

On note que la présence d'un composé de formule générale (I) permet d'améliorer significativement l'expression de l'activité luciferase d'un facteur variant de 10 à 15.

### Exemple 2 :

### Transfert de gène codant pour la β-galactosidase dans un muscle squelettique

De la même façon qu'en exemple 1, il est procédé à une injection, dans le tibial anterior de souris, d'ADN nu (A), de formulations conformes à l'invention contenant 5% (B) ou 10% de composé 304 (C), 0,01% (D) et 0,1% de composé 904 (E).

Sept jours après les injections, l'expression de la β-galactosidase est constatée par visualisation macroscopique des muscles ayant reçu les formulations conformes à la présente invention. Elles présentent des zones de fibres exprimant la β-galactosidase et qui deviennent bleues après révélation en plongeant les muscles dans une solution contenant 2mM de MgCl₂, du ferricyanide de potassium, du ferrocyanide de potassium, 5 mM de PBS, à pH 7 et en présence de 1 mg/ml de 5-bromo-4-chloro-3-indoyl-β-d-galactopyranoside. Les muscles sont photographiés 24h après incubation dans cette solution à 37°C. Les muscles n'ayant reçu que l'ADN nu ne permettent pas de visualiser une expression de la β-galactosidase.

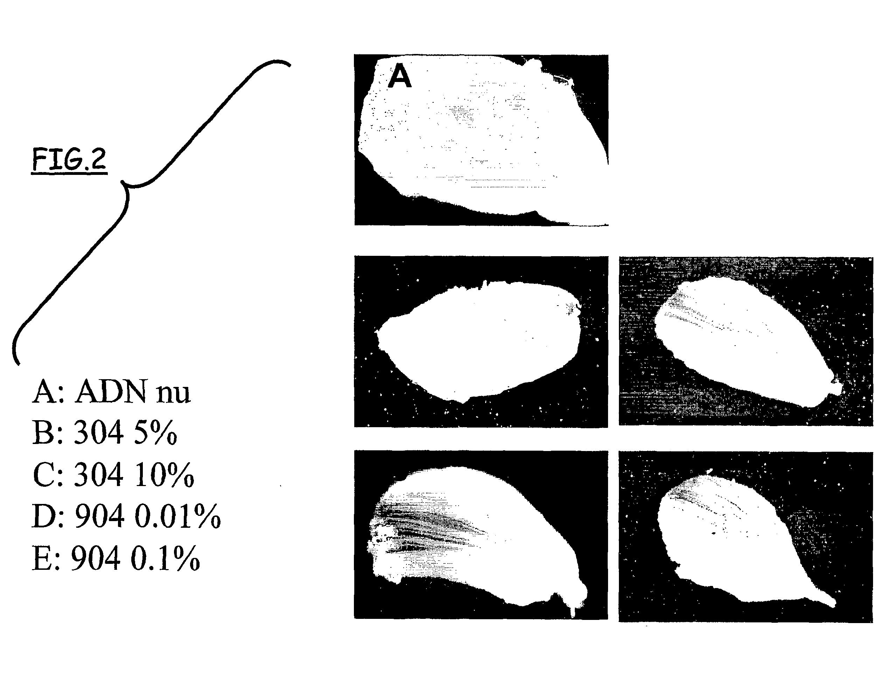

La figure 2 illustre ces résultats.

### Exemple 3 :

### Analyse histologique de l'expression de la Green-Fluorescent Proteine (GFP) 7 jours après injection de 50 µg d'ADN nu (A) ou de 50 µg d'ADN formulé selon l'invention

Les formulations selon l'invention testées sont :
- le composé 304 à 5 % (pds/v) (B) ;
- le composé 704 à 0,25 % (pds/v) (C) ;
- le composé 904 à 0,1 % (pds/v) (D) et
- le composé 908 à 10⁻³ % (pds/v).

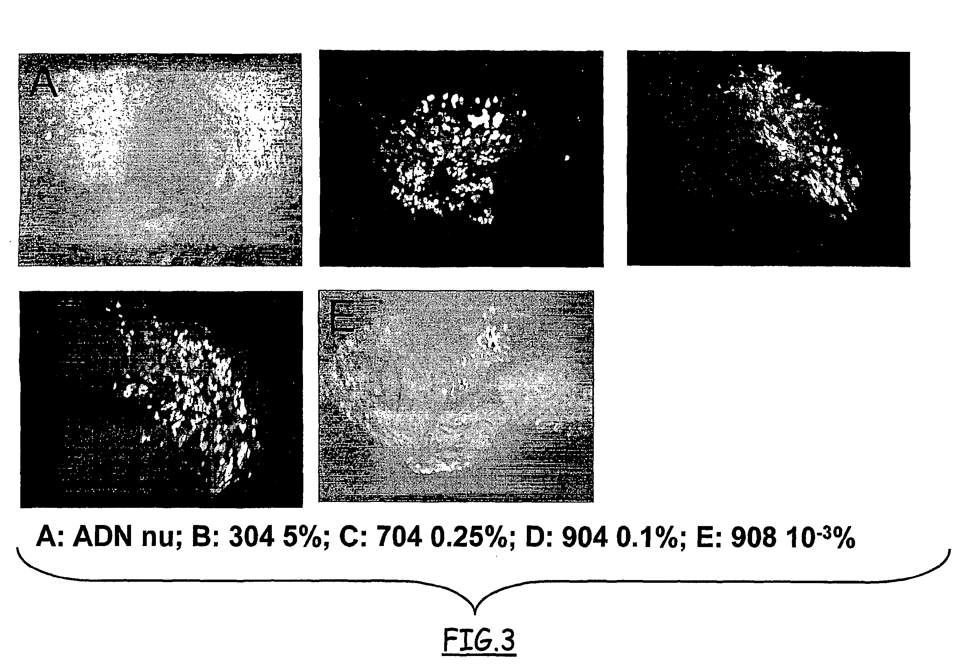

L'expression de la GFP est constatée sept jours après l'injection des formulations par l'observation d'une coupe de tissu montée entre lame et lamelle, puis examinée par microscopie de fluorescence. La figure 3 permet de constater que le nombre de fibres musculaires exprimant la GFP est beaucoup plus important lorsque l'ADN est formulé avec du 304 à 5 % (B), 704 à 0,25 % (C) et 904 à 0,1 % (D). Dans le cas particulier de l'ADN formulé avec du 908 à 10⁻³ % (E), il est constaté que le nombre de fibres transfectées est équivalent à celui obtenu avec l'ADN nu.

Les formulations conformes à la présente invention permettent par conséquent une transfection au moins aussi efficace, voire nettement supérieure, que l'ADN nu. Ces résultats confirment l'intérêt des formulations revendiquées de l'expression d'une protéine d'intérêt thérapeutique local ou systémique.

### Exemple 4 :

### Comparaison de l'efficacité de transfection dans le muscle des formulations conformes à la présente invention vis-à-vis de l'électrotransfert

Concernant la condition électrotransfert, 50 µg d'ADN codant pour la β-galactosidase ont été injectés dans le labial cranial de souris Swiss agées de 8 semaines, puis le muscle est soumis aux conditions ci-dessous. Pour ce qui est des formulations, 50 µg d'ADN PCMV-β gal ont été mélangés avec du 304, 704 et 904 puis injectés dans les libials cranials des souris.

La technique de l'électrotransfert est une des seules méthodes non virales qui permette l'amélioration de la transfection in vivo dans le muscle squelettique.

La technique de l'électrotransfert considérée dans le cadre de ce test comparatif consiste à soumettre un muscle à un champ électrique de 200 V/cm avec 8 impulsions de 20 ms à une fréquence de 2 Hz. Ce test est validé à travers l'expression de la β-galactosidase dans le muscle.

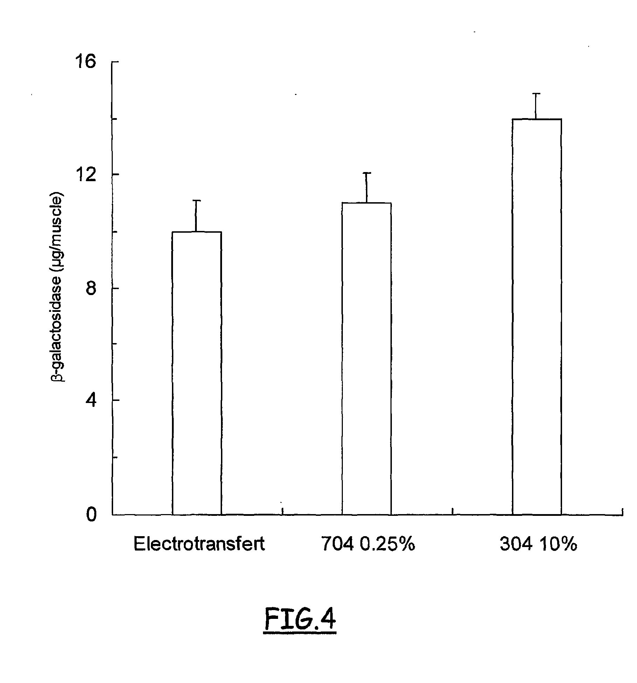

La figure 4 présente les résultats obtenus. On constate que l'injection d'ADN nu dans le muscle soumis à l'électrotransfert permet d'obtenir 7 jours après l'injection 10 µg β-galactosidase synthétisé par le muscle. Les formulations ADN 304 à 10 % (pds/v), et d'ADN 704 à 0,25 % (pds /v), permettent d'avoir la synthèse respectivement de 14 et 11 µg de β galactosidase/muscle.

Les formulations de la présente invention permettent d'obtenir au moins la même efficacité de transfection, voire plus, que celle de l'ADN électrotransféré. Qui plus est, la présente invention a pour net avantage de ne nécessiter aucun équipement particulier et d'être beaucoup plus simple à mettre en oeuvre puisqu'il suffit de mélanger le composé (I) avec l'ADN plasmidique. Enfin, il importe de souligner que l'électrotransfert ne peut pas être appliqué pour le transfert de gène dans le muscle cardiaque, contrairement aux formulations conformes à la présente invention.

L'électrotransfert est de plus une technique douloureuse et nécessite une anesthésie au moins régionale sinon générale.

### Exemple 5 :

### Morphologie, visualisée par cryo-microscopie électronique, des particules poloxamine/ADN en fonction du milieu de formulation

Des particules poloxamine 904/ADN ont été formulées soit avec du NaCl (150 mM), soit avec du Tyrode (milieu contenant 3 mM CaCl₂, 2 mM MgCl₂, 6 mM KCl, 140 mM NaCl, 10 mM glucose et 10 mM Hepes pH 7.4).

Par exemple, la mesure du potentiel zeta de la poloxamine 904 dans le Tyrode indique une valeur de +9,5 mV ce qui atteste de la mise en oeuvre cationique de la poloxamine lors de son association avec l'ADN en présence de Tyrode.

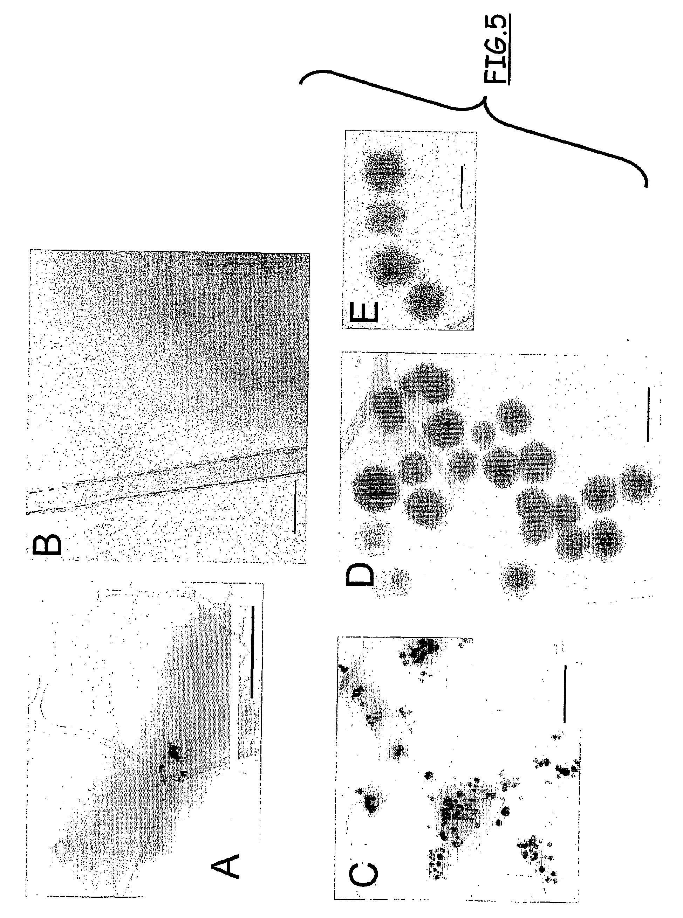

La morphologie des particules a ensuite été visualisée par cryo-microscopie électronique. La figure 5 rend compte de la visualisation.

Formulées avec du NaCl (150 mm), les molécules d'ADN s'agrègent les unes aux autres en présence de poloxamines 904 pour former des structures assez «lâches » de grande taille, alors qu'avec du Tyrode, les molécules d'ADN forment avec les poloxamines 904 des petites particules sphériques (figures 5C, D et E). La même observation peut être réalisée avec la poloxamine 304.

Ainsi, en contrôlant le pH et la composition ionique du milieu et notamment la présence de CaCl₂, des particules sphériques de petites tailles sont générées et auraient vraisemblablement un pouvoir de diffusion accrue dans les tissus.

### Exemple 6 :

### Influence du milieu de formulation sur l'efficacité de transfection des formulations poloxamine/ADN dans du NaCI ou du Tyrode

Chez des souris Swiss âgées de 8 semaines, 50 µl de formulations contenant 15 µg d'ADN plasmidique codant pour la luciférase sont injectés dans le tibial anterior. Les muscles sont prélevés 7 jours plus tard, broyés et l'activité luciferase est dosée.

Le tableau suivant montre que la quantité de protéine produite par le muscle squelettique est bien supérieure quand les formulations sont réalisées dans du Tyrode.

| Poloxamine | Milieu de formulation | Luciferase (ng/muscle) |
|---|---|---|
| 704 0.5% (p/v) | NaCl | 94 |
| 704 0.5% (p/v) | Tyrode | 625 |
| 904 0.01% (p/v) | NaCl | 74 |
| 904 0.01 % (p/v) | Tyrode | 971 |
| 904 0.1 % (p/v) | NaCl | 78 |
| 904 0.1 % (p/v) | Tyrode | 519 |

### Exemple 7 :

### Transfert in vivo dans le coeur d'un gène codant pour la β-galactosidase en présence d'un composé de formule générale (I)

### Préparation des échantillons :

Chaque essai met en oeuvre 200 µg d'ADN plasmidique contenant le gène codant pour la β-galactosidase et un polymère dans 700 µl de Tyrode.

Les polymères PE 6400, pour l'échantillon témoin et les poloxamines 304, 704 et 904 ont été testés aux concentrations (en poids/volume) de 0,5 %, 2,5 %, 0,75 % et 0,1 % respectivement.

Chaque échantillon ainsi formulé est injecté dans le sac péricardique de rats adultes d'environ 200 g après avoir traversé le diaphragme. Les coeurs sont prélevés 7 jours après l'injection et sont analysés par histologie pour révéler l'activité β-galactosidase. Seules les formulations à base de poloxamine permettent de transfecter des cardiomyocytes dans le ventricule gauche et notamment la poloxamine 304 qui donne les meilleurs résultats. Il a été observé avec la poloxamine 304 que des cardiomyocytes exprimant la β-galactosidase et donc colorés en bleu étaient présents jusqu'à dans 2/3 de la profondeur de la masse ventriculaire, ce qui montre leur pouvoir de diffusion important.

## Revendications

1. Composition pharmaceutique, **caractérisée en ce qu'**elle associe à au moins un acide nucléique au moins un sel minéral du copolymère tétrafonctionnel de formule (I) 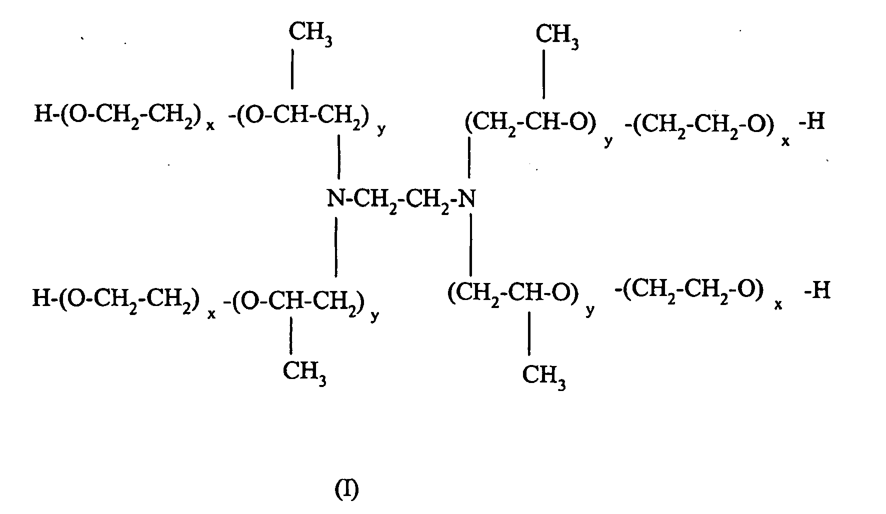 mis en oeuvre sous une forme cationique
dans laquelle x et y représentent indépendamment l'un de l'autre un entier compris entre 1 et 500 avec x possédant une valeur telle que ladite molécule comprend au moins 30 % en poids de motifs oxyde d'éthylène, la dite composition étant formulée avec du milieu Tyrode, à savoir 140 mM NaCl, 6 mM KCI, 3 mM CaCl₂, 2 mM MgCl₂, 10 mM Hepes pH 7.4 et 10 mM glucose.

2. Composition selon la revendication précédente, **caractérisée en ce que** le composé de formule générale (I) possède au plus 85 % en poids de motifs oxyde d'éthylène.

3. Composition selon la revendication 1 ou 2, **caractérisée en ce que** le composé de formule générale (I) possède entre 35 et 50 % en poids de motifs oxyde d'éthylène.

4. Composition selon l'une des revendications précédentes, **caractérisée en ce que** le composé de formule générale (I) possède un poids moléculaire d'au moins 800 g/mol et plus préférentiellement compris entre 1 000 et 25 000g/mol.

5. Composition selon l'une des revendications précédentes, **caractérisée en ce que** le rapport OE/OP est compris entre 0,5 et 1,5, et de préférence de l'ordre de 1 ± 0,2.

6. Composition selon l'une des revendications précédentes, **caractérisée en ce que** ladite molécule est choisie parmi les molécules possédant un poids moléculaire de 1 650 g/mol avec un rapport OE/OP de 15 :16, un poids moléculaire de 5 500 g/mol pour un rapport OE/OP 50:56 et un poids moléculaire de 6 700 g/mol pour un rapport OE/OP 61:68.

7. Composition selon l'une des revendications précédentes, **caractérisée en ce que** le composé de formule générale (I) est présent à raison de 0,005 % à 15 % en poids/volume et plus préférentiellement entre 0,01 % et 10 % en poids/volume.

8. Composition selon l'une des revendications précédentes, **caractérisée en ce que** le composé de formule générale (I) comprend en outre au moins un motif de ciblage intra- ou extracellulaire choisi parmi un anticorps, un ligand de récepteur membranaire, une lectine, une protéine à motif RGD.

9. Composition selon l'une des revendications précédentes, **caractérisée en ce que** l'acide nucléique est un acide désoxyribonucléique.

10. Composition selon l'une des revendications 1 à 8, **caractérisée en ce que** l'acide nucléique est un acide ribonucléique.

11. Composition selon l'une des revendications 1 à 10, **caractérisée en ce que** l'acide nucléique est modifié chimiquement.

12. Composition selon l'une des revendications 1 à 8, **caractérisée en ce que** l'acide nucléique est un antisens.

13. Composition selon l'une des revendications 1 à 8, **caractérisée en ce que** l'acide nucléique est un ARN interférent.

14. Composition selon l'une des revendications précédentes, **caractérisée en ce que** l'acide nucléique comporte un gène thérapeutique.

15. Composition pharmaceutique selon l'une des revendications 1 à 14 préparée par un mélange volume à volume, d'une solution deux fois concentrée contenant l'acide nucléique et du Tyrode et une solution aqueuse deux fois concentrée contenant le copolymère tétrafonctionnel.

16. Utilisation d'une composition selon l'une des revendications 1 à 15 pour la préparation d'une composition destinée à assurer le transfert cellulaire *in vivo* d'au moins un acide nucléique.

17. Utilisation selon la revendication 16, dans laquelle le transfert *in vivo* est un transfert dans des cellules musculaires.

18. Utilisation selon la revendication 16, dans laquelle le transfert *in vivo* est un transfert dans des cellules cardiaques.

19. Utilisation selon la revendication 18, **caractérisée en ce que** le composé de formule (I) est la poloxamine 304 formulée avec du milieu Tyrode, à savoir 140 mM NaCl, 6mM KCI, 3 mM CaCl₂, 2mM MgCl₂, 10 mM Hepes pH 7.4 et 10 mM glucose.

## Claims

1. A pharmaceutical composition, **characterized in that** it combines with at least one nucleic acid at least one mineral salt of the tetra-functional copolymer of formula (I): 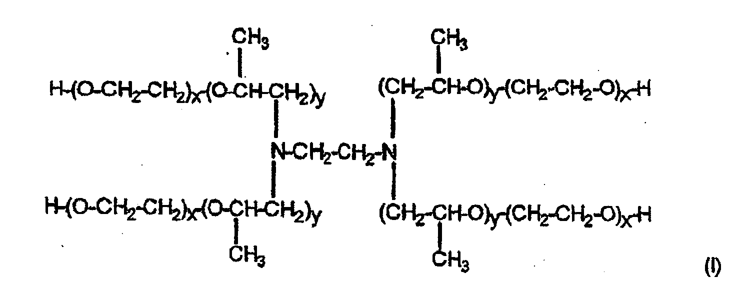 used in a cationic form,
in which x and y represent, independently of one another, an integer of between 1 and 500 with x having a value such that said molecule comprises at least 30% by weight of ethylene oxide units, said composition being formulated with Tyrode's medium, i.e. 140 mM NaCl, 6 mM KC1, 3 mM CaCl₂, 2 mM MgCl₂, 10 mM Hepes, pH 7.4, and 10 mM glucose.

2. The composition as claimed in the preceding claim, **characterized in that** the compound of general formula (I) has no more than 85% by weight of ethylene oxide units.

3. The composition as claimed in claim 1 or 2, **characterized in that** the compound of general formula (I) has between 35 and 50% by weight of ethylene oxide units.

4. The composition as claimed in one of the preceding claims, **characterized in that** the compound of general formula (I) has a molecular weight of at least 800 g/mol, and more preferably of between 1000 and 25 000 g/mol.

5. The composition as claimed in one of the preceding claims, **characterized in that** the EO/PO ratio is between 0.5 and 1.5, and preferably of the order of 1 ± 0.2.

6. The composition as claimed in one of the preceding claims, **characterized in that** said molecule is chosen from the molecules having a molecular weight of 1650 g/mol with an EO/PO ratio of 15:16, a molecular weight of 5500 g/mol for an EO/PO ratio of 50:56, and a molecular weight of 6700 g/mol for an EO/PO ratio of 61:68.

7. The composition as claimed in one of the preceding claims, **characterized in that** the compound of general formula (I) is present in a proportion of 0.005% to 15% by weight/volume, and more preferably between 0.01% and 10% by weight/volume.

8. The composition as claimed in one of the preceding claims, **characterized in that** the compound of general formula (I) also comprises at least one intra- or extracellular targeting unit selected from an antibody, a membrane-receptor ligand, a lectin, a protein containing a RDG unit.

9. The composition as claimed in one of the preceding claims, **characterized in that** the nucleic acid is a deoxyribonucleic acid.

10. The composition as claimed in one of claims 1 to 8, **characterized in that** the nucleic acid is a ribonucleic acid.

11. The composition as claimed in one of claims 1 to 10, **characterized in that** the nucleic acid is chemically modified.

12. The composition as claimed in one of claims 1 to 8, **characterized in that** the nucleic acid is an antisense.

13. The composition as claimed in one of claims 1 to 8, **characterized in that** the nucleic acid is an interfering RNA.

14. The composition as claimed in one of the preceding claims, **characterized in that** the nucleic acid comprises a therapeutic gene.

15. The pharmaceutical composition as claimed in one of claims 1 to 14, prepared by mixing volume for volume a two-times concentrated solution containing the nucleic acid and Tyrode's and a two-times concentrated aqueous solution containing the tetrafunctional copolymer.

16. A use of a composition as claimed in one of claims 1 to 15, for preparing a composition intended to provide the cellular transfer in vivo of at least one nucleic acid.

17. The use as claimed in claim 16, in which the *in vivo* transfer is a transfer into muscle cells.

18. The use as claimed in claim 16, in which the in vivo transfer is a transfer into cardiac cells.

19. The use as claimed in claim 18, **characterized in that** the compound of formula (I) is poloxamine 304 formulated with Tyrode's medium, i.e. 140 mM NaCl, 6 mM KCl, 3 mM CaCl₂, 2 mM MgCl₂, 10 mm Hepes, pH 7.4, and 10 mM glucose.

## Patentansprüche

1. Arzneimittelzusammensetzung, **dadurch gekennzeichnet, dass** in ihr mindestens eine Nukleinsäure mit mindestens einem Mineralsalz eines tetrafunktionellen Copolymeren der Formel (I) kombiniert ist 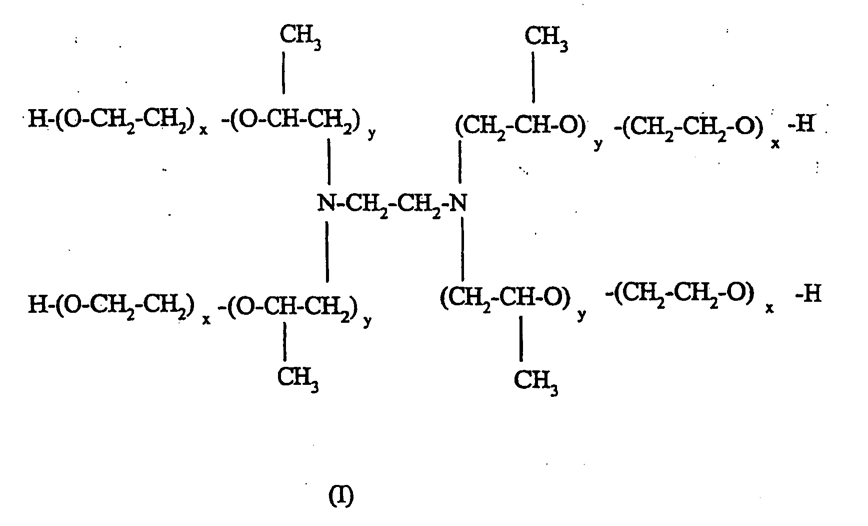 das in kationischer Form vorliegt,
in der x und y unabhängig voneinander eine ganze Zahl von 1 bis 500 darstellen, wobei x einen solchen Wert hat, dass das Molekül mindestens 30 Gew.-% Ethylenoxid-Einheiten enthält, wobei die Zusammensetzung mit einem Tyrode-Medium, d.h. 140 mM NaCl, 6 mM KCl, 3 mM CaCl₂, 2 mM MgCl₂, 10 mM Hepes pH 7,4 und 10 mM Glucose formuliert ist.

2. Zusammensetzung nach dem vorhergehenden Patentanspruch, **dadurch gekennzeichnet, dass** die Verbindung der allgemeinen Formel (I) höchstens 85 Gew.-% Ethylenoxid-Einheiten enthält.

3. Zusammensetzung nach Anspruch 1 oder 2, **dadurch gekennzeichnet, dass** die Verbindung der allgemeinen Formel (I) zwischen 35 und 50 Gew.-% Ethylenoxid-Einheiten enthält.

4. Zusammensetzung nach einem der vorhergehenden Ansprüche, **dadurch gekennzeichnet, dass** die Verbindung der allgemeinen Formel (I) ein Molekulargewicht von mindestes 800 g/Mol und vorzugsweise zwischen 1000 und 25 000 g/Mol aufweist.

5. Zusammensetzung nach einem der vorhergehenden Ansprüche, **dadurch gekennzeichnet, dass** das Verhältnis OE/OP zwischen 0,5 und 1,5 und vorzugsweise in der Größenordnung von 1±0,2 liegt.

6. Zusammensetzung nach einem der vorhergehenden Ansprüche, **dadurch gekennzeichnet, dass** das besagte Molekül unter Molekülen ausgewählt ist, die ein Molekulargewicht von 1650 g/Mol mit einem Verhältnis OE/OP von 15:16, ein Molekulargewicht von 5500 g/Mol für ein Verhältnis OE/OP von 50:56 und ein Molekulargewicht von 6700 g/Mol für ein Verhältnis OE/OP von 61:68 haben.

7. Zusammensetzung nach einem der vorhergehenden Ansprüche, **dadurch gekennzeichnet, dass** die Verbindung der allgemeinen Formel (I) in einer Menge von 0,005 % bis 15 % (Gewicht/Volumen) und bevorzugt zwischen 0,01 % und 10 % (Gewicht/Volumen) vorliegt.

8. Zusammensetzung nach einem der vorhergehenden Ansprüche, **dadurch gekennzeichnet, dass** die Verbindung der allgemeinen Formel (I) außerdem mindestens ein intra- oder extrazelluläres Target enthält, das unter Antikörpern, Membranrezeptor-Liganden, Lectinen und Proteinen mit RGD-Einheiten ausgewählt ist.

9. Zusammensetzung nach einem der vorhergehenden Ansprüche, **dadurch gekennzeichnet, dass** die Nukleinsäure einen Desoxyribonukleinsäure ist.

10. Zusammensetzung nach einem der Ansprüche 1 bis 8, **dadurch gekennzeichnet, dass** die Nukleinsäure eine Ribonukleinsäure ist.

11. Zusammensetzung nach einem der Ansprüche 1 bis 10, **dadurch gekennzeichnet, dass** die Nukleinsäure chemisch modifiziert ist.

12. Zusammensetzung nach einem der Ansprüche 1 bis 8, **dadurch gekennzeichnet, dass** die Nukleinsäure eine mit Antisinn ist.

13. Zusammensetzung nach einem der Ansprüche 1 bis 8, **dadurch gekennzeichnet, dass** die Nukleinsäure ein ARN-Interferent ist.

14. Zusammensetzung nach einem der vorhergehenden Ansprüche, **dadurch gekennzeichnet, dass** die Nukleinsäure ein therapeutisches Gen enthält.

15. Arzneimittelzusammensetzung nach einem der Ansprüche 1 bis 14, hergestellt durch Vermischen einer Volumenmenge zu einer Volumenmenge einer zweifach konzentrierten Lösung, welche die Nukleinsäure und Tyrode-Medium enthält, und einer wässerigen zweifach konzentrierten Lösung, die das tetrafunktionelle Copolymere enthält.

16. Verwendung einer Zusammensetzung nach einem der Ansprüche 1 bis 15 zur Herstellung einer Zusammensetzung, die zur Gewährleistung des Zelltransfers mindestens einer Nukleinsäure in vivo bestimmt ist.

17. Verwendung nach Anspruch 16, wobei der Transfer in vivo ein Transfer in Muskelzellen ist.

18. Verwendung nach Anspruch 16, wobei der Transfer in vivo ein Transfer in Herzzellen ist.

19. Verwendung nach Anspruch 18, **dadurch gekennzeichnet, daß** die Verbindung der Formel (I) Poloxamin 304 ist, welches mit Tyrode-Medium, d.h. 140 mM NaCl, 6mM KC1, 3mM CaCl₂, 2 mM MgCl₂, 10mM Hepes pH 7,4 und 10 mM Glucose formuliert ist.
